# EUROPÄISCHE PATENTSCHRIFT

(11) **EP 1 481 371 B1**
(45) Veröffentlichungstag und Bekanntmachung des Hinweises auf die Patenterteilung: **24.08.2005**
(21) Anmeldenummer: 02807249.4
(22) Anmeldetag: 11.09.2002
(51) Int. Cl.: G06T 7/00, G06K 9/00

(54) **VERFAHREN ZUR TRENNUNG EINER IN EINEM BILD EINER PROBE ENTHALTENEN ZELLGRUPPE IN EINZELZELLEN**
METHOD FOR SEPARATING A GROUP OF CELLS WHICH ARE CONTAINED IN AN IMAGE SAMPLE INTO INDIVIDUAL CELLS
PROCEDE PERMETTANT DE SEPARER EN CELLULES INDIVIDUELLES UN GROUPE DE CELLULES, CONTENU DANS UNE IMAGE D'UN PRELEVEMENT

(30) Priorität: 22.04.2002 DE 10217858
(43) Veröffentlichungstag der Anmeldung: 01.12.2004
(73) Patentinhaber: Fraunhofer-Gesellschaft zur Förderung der angewandten Forschung e.V., 80636 München (DE)
(72) Erfinder: WITTENBERG, Thomas, 91054 Erlangen (DE); GROBE, Matthias, 90491 Nürnberg (DE); COURONNE-SCHMIDT, Robert, 91058 Erlangen (DE); KUZIELA, Heiko, 91052 Erlangen (DE); MUENZENMAYER, Christian, 90408 Nürnberg (DE); SPINNLER, Klaus, 91056 Erlangen (DE); DIETRICH, Paulus, 91074 Herzogenaurach (DE)
(74) Vertreter: Schoppe, Fritz
(86) Internationale Anmeldenummer: PCT/EP2002/010200
(87) Internationale Veröffentlichungsnummer: WO 2003/090169

(56) Entgegenhaltungen:
- EP-A- 0 014 857
- WO-A-96/09594
- WO-A-96/09604
- US-A- 5 987 158
- MACKIN R W JR ET AL: "Adaptive 3-D segmentation algorithms for microscope images using local in-focus, and contrast features: application to Pap smears" PROCEEDINGS OF THE INTERNATIONAL CONFERENCE ON IMAGE PROCESSING. (ICIP). WASHINGTON, OCT. 23 - 26, 1995, LOS ALAMITOS, IEEE COMP. SOC. PRESS, US, Bd. 3, 23. Oktober 1995 (1995-10-23), Seiten 160-163, XP010197155 ISBN: 0-7803-3122-2

## Beschreibung

Die vorliegende Erfindung bezieht sich auf ein Verfahren zur Trennung einer in einer Probe enthaltenen Zellgruppe in Einzelzellen, insbesondere bezieht sich die vorliegende Erfindung auf ein Verfahren zur Trennung einer in einem Bild einer Probe enthaltenen Zellgruppe in Einzelzellen für eine nachfolgende Klassifikation der Probe, wobei die Zellgruppen eine Mehrzahl einander überlappender Zellen aufweist.

Zur erfolgreichen Behandlung von Krebserkrankungen ist eine frühzeitige Erkennung und Behandlung erforderlich. Dies kann durch den regelmäßigen Besuch von Krebs-Vorsorgeuntersuchungen erreicht werden. Bei solchen Untersuchungen werden Abstriche des zu untersuchenden Gewebes genommen, wobei im Falle einer Untersuchung auf Gebärmutterkrebs (Zervix Karzinom) dies üblicherweise durch den "PAP-Test" erfolgt, benannt nach dem griechischen Arzt Dr. George Papanicolaou, welcher dieses Verfahren 1942 einführte. Dieser gynäkologische Abstrich der Zervix, d. h. des Gebärmutterhalses, oder Zellpräparate, die von anderen Untersuchungen erhalten wurden, allgemein als zytologisches Präparat bezeichnet, müssen klassifiziert werden. Hierzu werden die zytologischen Präparate auf einen Objektträger aufgetragen, gefärbt und durch eine zytologische Fachkraft unter dem Mikroskop beurteilt.

Zur objektiven Diagnoseunterstützung einer solchen Fachkraft werden in jüngster Zeit Bildverarbeitungsprogramme verwendet, mit denen die Zellen eines Präparats automatisch segmentiert und aufgrund der morphometrischen Eigenschaften von Zellkern und Zellplasma, z. B. Ausdehnung des Kerns und des Plasmas, Form des Kerns und des Plasmas, relative Größe von Kern und Plasma zueinander, etc., sowie aufgrund der Texturierung der Chromatinstruktur in dem Zellkern klassifiziert werden. Die hier eingesetzten Bildverarbeitungsprogramme ermöglichen jedoch nur dann eine zuverlässige Segmentierung der Zellen eines Präparats, wenn diese Zellen des Präparats vereinzelt vorliegen.

Während zytologische Fachkräfte aufgrund ihrer Ausbildung in der Lage sind, implizit sich bis zu einem gewissen Grade gegenseitig überlappende Zellplasmen voneinander zu trennen, um anschließend daraus eine Diagnose in gesunde, entzündliche, dysplastische oder kranke Zellen vorzunehmen s. z.B. US 5 987 158, sind automatische Verfahren zur Segmentierung und Trennung von solchen überlappenden Zellen nicht bekannt.

Herkömmlicherweise werden für automatisierte Klassifikations-Ansätze entweder ausschließlich vereinzelte Zellen verwendet oder zwischen den automatisierten Schritten wird eine manuelle Zellteilung eingeschoben.

Der Nachteil des ersten Ansatzes, der Verwendung von nur vereinzelten Zellen, besteht darin dass hier die überlappenden Zellen nicht weiter beachtet werden, also verworfen werden, so dass die auch in den überlappenden Zellen enthaltenen wichtigen Informationen zur Klassifikation einer Probe verloren gehen.

Dieser Verlust an Information wird zwar bei dem zweiten Ansatz vermieden, jedoch besteht der Nachteil hier darin, dass keine vollautomatische Vorbeurteilung einer Probe möglich ist. Sobald eine Überlappung angetroffen wird oder wahrscheinlich ist, ist es erforderlich einen menschlichen Begutachter für die Trennung heranzuziehen. Nach der Trennung geht dann das automatische Verfahren weiter.

Das Vorliegen vereinzelter Zellen ist im Regelfall jedoch die Ausnahme bei zytologischen Präparaten. Vielmehr werden sich in den zytologischen Präparaten/Proben, je nach Präparationsart, bis zu 80% aller Zellplasmen eines Präparats gegenseitig überdecken. Somit ist es für fast jede Klassifikation von zytologischen Präparaten erforderlich, eine manuelle Zellteilung vorzusehen oder auf die in diesen überlappenden Zellen enthaltenen Informationen zu verzichten.

Ausgehend von dem oben beschriebenen Stand der Technik liegt der vorliegenden Erfindung daher die Aufgabe zugrunde, ein Verfahren zu schaffen, das eine Überdeckung von Zellen oder Zellplasmen erkennt, und die einzelnen, überlappenden Zellen segmentiert und trennt, um so die Erfordernisse für eine automatische Klassifikation/Beurteilung einer Probe zu ermöglichen.

Diese Aufgabe wird durch ein Verfahren nach Anspruch 1 gelöst.

Die vorliegende Erfindung schafft ein Verfahren zur Trennung einer in einem Bild einer Probe enthaltenen Zellgruppe in Einzelzellen für eine nachfolgende Klassifikation der Probe, wobei die Zellgruppe eine Mehrzahl einander überlappender Zellen aufweist, mit folgenden Schritten:
(a) Auswählen eines Zellkerns einer ersten Zelle, die aus der Zellgruppe herauszutrennen ist, wobei der Zellkern der ersten Zelle benachbart zu einem Zellkern der zweiten Zelle angeordnet ist, wobei das Zellplasma der ersten Zelle und das Zellplasma der zweiten Zelle einander derart überlappen, dass ein gemeinsames Zellplasma gebildet ist;
(b) Bestimmen einer Einschnürung des gemeinsamen Zellplasmas zwischen dem Zellkern der ersten Zelle und dem Zellkern der zweiten Zelle;
(c) Trennen des gemeinsamen Zellplasmas an der Einschnürung;
(d) Festlegen eines Bereichs des gemeinsamen Zellplasmas, in dem die Überlappung der Zellplasmen der ersten Zelle und der zweiten Zelle erwartet wird;
(e) Klassifizieren des festgelegten Bereichs, um einzelne Abschnitte desselben zu dem Zellplasma der ersten Zelle und/oder zu dem Zellplasma der zweiten Zelle zuzuordnen; und
(f) Vervollständigen des im Schritt (c) erhaltenen Zellplasmas der ersten Zelle basierend auf den klassifizierten Abschnitten.

Gemäß einem bevorzugten Ausführungsbeispiel der vorliegenden Erfindung basiert das Verfahren auf einem Bild, welches von einem zytologischen Präparat bzw. einer Probe erzeugt wurde. Das Bild wurde beispielsweise mittels eines Mikroskops erzeugt und digitalisiert, wobei jedes Bild eine von der Aufnahmevorrichtung (Kamera, Objektiv, etc.) abhängige Auflösung, beispielsweise 1000 x 700 Pixel, aufweist. Das Bild wurde entweder in der Durchlichtmodalität oder in der Fluoreszenzmodalität aufgenommen, wobei andere, bekannte Aufnahmemodalitäten auch herangezogen werden können. Gemäß einem anderen Ausführungsbeispiel wird anstelle eines Bildes eine Mehrzahl von Bildern verwendet, welche miteinander registriert sind, und die bei unterschiedlichen Aufnahmemodalitäten erzeugt wurden. Die unterschiedlichen Aufnahmemodalitäten umfassen beispielsweise die Aufnahme eines Bilds in einer Durchlichtaufnahmemodalität und die Aufnahme eines weiteren Bildes in einer Fluoreszenzaufnahmemodalität. Alternativ können die Bilder auch beide in der Fluoreszenzaufnahmemodalität jedoch mit unterschiedlichen Parametern hinsichtlich der Fluoreszenz erzeugt werden.

Auf der Grundlage der so erzeugten Bilder erfolgt unter Ausnutzung des erfindungsgemäßen Verfahrens eine automatische Segmentierung oder Trennung von Zellgruppen in Einzelzellen, die dann einer automatisierten Weiterverarbeitung zur Klassifikation der zytologischen Probe zugrunde gelegt werden können.

Der Vorteil der vorliegenden Erfindung besteht somit darin, dass auf die Zwischenschaltung einer manuellen Zellteilung und den damit verbundenen Arbeitsaufwand und den Zeitverlust verzichtet werden kann und gleichzeitig die in den Zellgruppen, auch als Zellhaufen bezeichnet, enthaltenen Informationen zur Klassifikation von zytologischen Präparaten nicht mehr verloren gehen, sondern zur Klassifikation derselben herangezogen werden, um so die Ergebnisse der Klassifikation auf eine breitere Basis der in dem zytologischen Präparat enthaltenen Zellen stellen zu können. Hierdurch ergibt sich der Vorteil einer Verbesserung der Zuverlässigkeit der nachfolgend durchgeführten Klassifikation der Präparate.

Gemäß einem weiteren bevorzugten Ausführungsbeispiel umfasst das erfindungsgemäße Verfahren auch noch die erforderlichen Vorbereitungsschritte, um aus einer Aufnahme (ein Bild oder mehrere Bilder) der Probe eine oder mehrere Zellgruppen zu erkennen, die dann erfindungsgemäß in Einzelzellen getrennt werden. Gemäß diesem Ausführungsbeispiel wird zunächst eine Erfassung und Segmentierung von Zellkernen anhand eines Bildes der Probe durchgeführt, um eine Liste von Zellkernen zu erzeugen. Anschließend erfolgt eine Erfassung und Segmentierung von Zellplasmen anhand des Bildes der Probe, um eine Liste der Zellplasmen zu erzeugen. Die Zellkerne werden den zugehörigen Zellplasmen zugeordnet, und anhand der Anzahl von Zellkernen, die einem Zellplasma zugeordnet wurden, wird erkannt, ob es sich bei der Kombination um einen Zellhaufen bzw. eine Zellgruppe oder eine segmentierte Einzelzelle handelt.

Bevorzugte Weiterbildungen der vorliegenden Erfindung sind in den Unteransprüchen definiert.

Nachfolgend werden anhand der beiliegenden Zeichnungen bevorzugte Ausführungsbeispiele der vorliegenden Erfindung näher erläutert. Es zeigen:
- Fig. 1: die einzelnen Verfahrensschritte zur Segmentierung einer erkannten Zellgruppe gemäß dem erfindungsgemäßen Verfahren zeigt;
- Fig. 2A bis 2E: die Bestimmung von benachbarten Zellkernen in einer Zellgruppe gemäß einem bevorzugten Ausführungsbeispiel;
- Fig. 3A bis 3C: die Lokalisierung von Einschnürungen gemäß einem bevorzugten Ausführungsbeispiel der vorliegenden Erfindung anhand von zwei benachbarten Zellkernen;
- Fig. 4A bis 4E: die Trennung eines gemeinsamen Zellplasmas gemäß einem bevorzugten Ausführungsbeispiel;
- Fig. 5A bis 5B: die Festlegung eines Bereichs des gemeinsamen Zellplasmas, in dem eine Überlappung der Zellplasmen vermutet wird, gemäß einem bevorzugten Ausführungsbeispiel;
- Fig. 6A bis 6E: die Vervollständigung einer herausgetrennten Zelle gemäß einem bevorzugten Ausführungsbeispiel; und
- Fig. 7: ein weiteres bevorzugtes Ausführungsbeispiel des erfindungsgemäßen Verfahrens zeigt, welches eine Erkennung von Zellgruppen aus einem Probenbild umfasst.

Hinsichtlich der nachfolgenden Beschreibung wird darauf hingewiesen, dass in den einzelnen Figuren ähnliche oder gleichwirkende Elemente mit gleichen Bezugszeichen versehen sind.

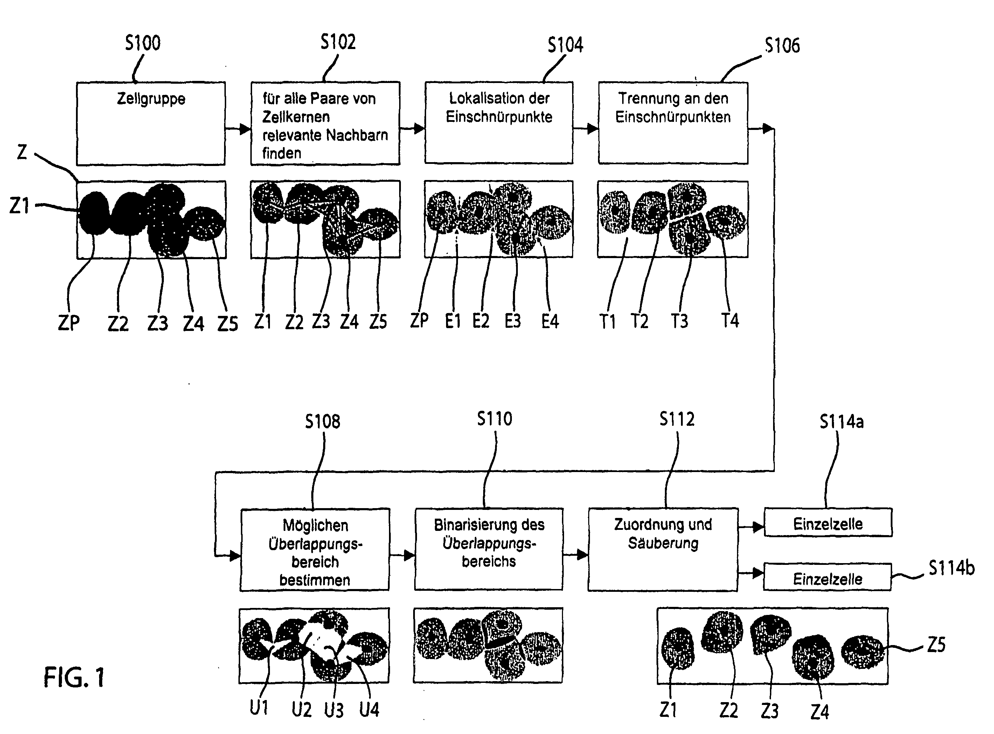

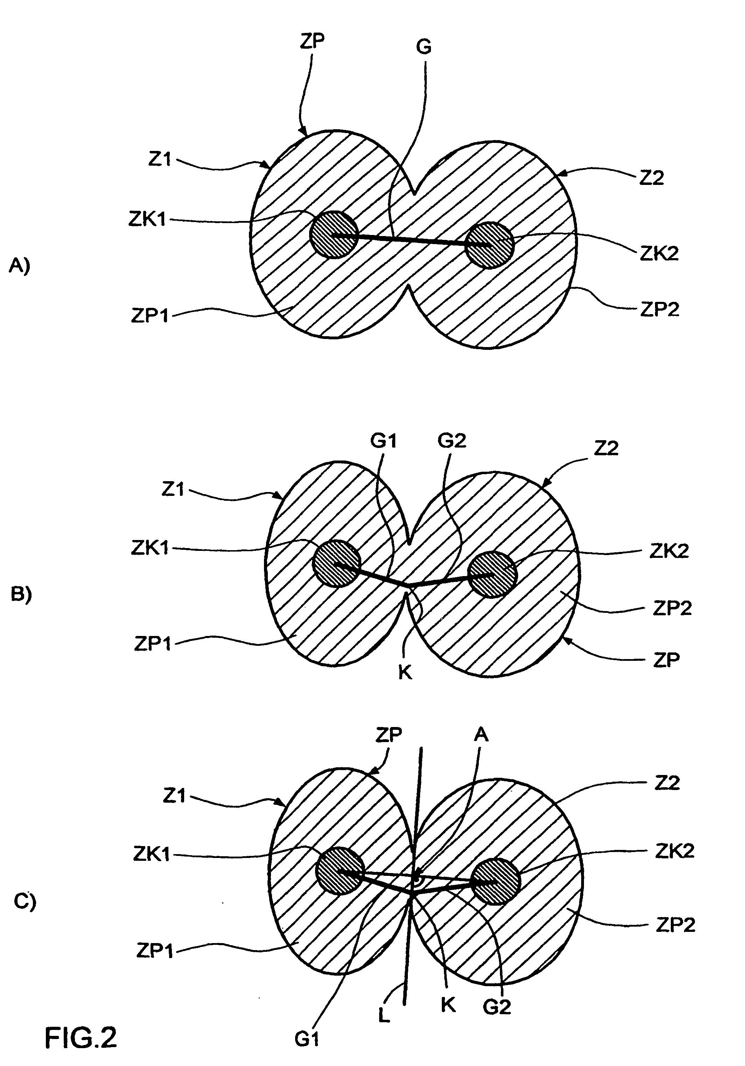

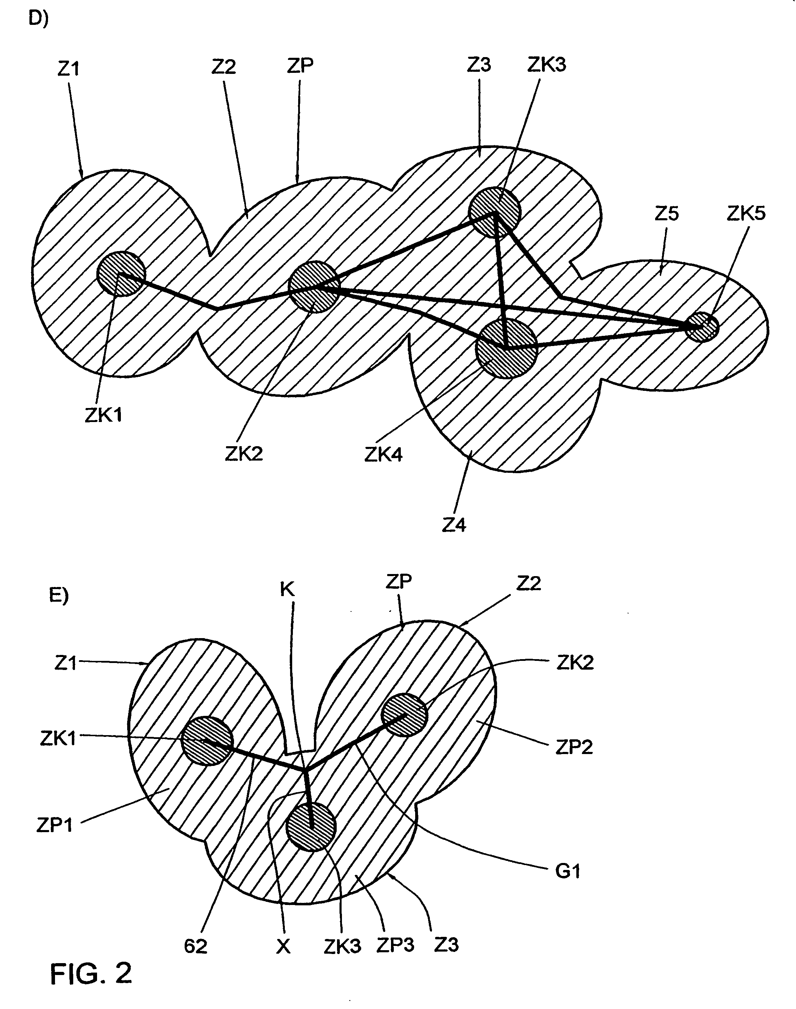

Anhand der Fig. 1 wird nachfolgend ein erstes, bevorzugtes Ausführungsbeispiel des erfindungsgemäßen Verfahrens näher erläutert. Fig. 1 zeigt ein Blockdiagramm, welches ausgehend von einer in einem Probenbild erfassten einzelnen Zellgruppe eine Segmentierung der Zellgruppe in Einzelzellen erfolgt. Bevorzugte Ausgestaltungen der anhand des Blockdiagramms in Fig. 1 beschriebenen Einzelschritte werden nachfolgend anhand der Fig. 2 bis 6 noch näher erläutert. In dem in Fig. 1 zugrundeliegenden Bild wurden ausgehend von einem ursprünglichen Probenbild bereits die zu erkennenden Zellkerne und Zellplasmen bestimmt, so dass die Flächen der Zellkerne und der Zellplasmen vorliegen, vorzugsweise als Binärmasken. Die Erkennung der in dem ursprünglichen Probenbild enthaltenen Zellkerne und Zellplasmen zur Erkennung eines Zellhaufens in dem Probenbild wird später noch näher beschrieben.

Fig. 1 zeigt die Trennung einer Zellgruppe, eines sogenannten Zellhaufens, also von Zellplasmen mit mehr als einem Zellkern, d. h. einander überlappende Zellen, in seine Bestandteile von einzelnen Zellen. In Fig. 1 werden zum einen die Einzelschritte gemäß dem bevorzugten erfindungsgemäßen Verfahren erläutert, wobei jedem der Einzelschritte eine schematische Darstellung des Zellhaufens nach dem Durchführen des entsprechenden Schrittes zugeordnet ist.

In Fig. 1 beginnt das Verfahren mit dem Schritt S100, in dem eine Zellgruppe Z mit einer Mehrzahl von Zellen Z1 bis Z5 bereitgestellt wird. Jede der Zellen Z1 bis Z5 umfasst einen Zellkern und ein Zellplasma. Bei der im Schritt S100 bereitgestellten Zellgruppe handelt es sich um eine bildliche Wiedergabe der Zellgruppe, die aus einer digitalisierten Aufnahme einer zu untersuchenden zytologischen Probe erzeugt wird, wie dies nachfolgend noch näher erläutert werden wird. Das erfindungsgemäße Verfahren basiert auf den in der Aufnahme der Zellgruppe enthaltenen Bildinformationen. Eine Modifikation der tatsächlichen zytologischen Probe bzw. des vorbereiteten zytologischen Präparats erfolgt nicht.

Nachdem die Zellgruppe im Schritt S100 bereitgestellt wurde, geht das Verfahren zum Schritt S102 weiter, in dem für alle Paare von Zellkernen relevante Nachbarn, d. h. relevante Nachbarzellkerne, erfasst werden. Ein Ausführungsbeispiel zur Auswahl oder Erfassung relevanter Nachbarzellen wird nachfolgend noch detaillierter beschrieben. Bei dem in Fig. 1 gezeigten allgemeinen Ausführungsbeispiel, sei angenommen, dass die Zellen Z1 und Z2 die erforderlichen Kriterien für das Vorliegen einer Nachbarschaft erfüllen, also die Zelle Z2 benachbart zu der herauszutrennenden Zelle Z1 ist. Ebenso sind die Zellen Z1 und Z3 zu der herauszutrennenden Zelle Z2 benachbart. Die Zellen Z2 und Z4 sind zu der herauszutrennenden Zelle Z3 benachbart. Die Zellen Z3 und Z5 sind zu der herauszutrennenden Zelle Z4 benachbart. Die Zelle Z4 ist zu der herauszutrennenden Zelle Z5 benachbart.

Nachdem die benachbarten Zellkerne im Schritt S102 festgestellt wurden, geht das Verfahren zum Schritt S104 weiter, in dem sogenannte Einschnürpunkte lokalisiert werden. Einschnürpunkte oder Einschnürungen sind Abschnitte des durch alle Zellplasmen ZP aller Zellen Z1 bis Z5 gebildeten gemeinsamen Zellplasmas, also Abschnitte des gemeinsamen Zellplasmas, bei denen eine Ausdehnung desselben, verglichen mit der üblichen Ausdehnung niedrig oder sogar minimal ist. Die Lokalisierung der Einschnürpunkte gemäß dem Schritt S104 erfolgt basierend auf einer Auswertung des gemeinsamen Plasmas, welches zwischen einer herauszutrennenden Zelle und einer zu der herauszutrennenden Zelle benachbarten Zelle liegt. Bei dem in Fig. 1 gezeigten Ausführungsbeispiel ergeben sich vier Einschnürungen E1 bis E4. Die Einschnürung E1 liegt zwischen der Zelle Z1 und der Zelle Z2 und wurde aufgrund einer Betrachtung dieser zwei Zellen festgestellt. Die übrigen Einschnürungen wurden ebenfalls durch eine Betrachtung der benachbarten Zellen festgestellt.

Nachdem die Einschnürpunkte bzw. Einschnürungen in dem gemeinsamen Plasma ZP festgestellt wurden, geht das Verfahren zum Schritt S106, in dem eine Trennung des gemeinsamen Zellplasmas basierend auf den im Schritt S104 lokalisierten Einschnürpunkten durchgeführt wird. Wie aus Fig. 1 zu erkennen ist, wurde das gemeinsame Plasma an den Positionen T1 bis T4 aufgetrennt, so dass die Zellen Z1 bis Z5 in dem Bild nun getrennt voneinander vorliegen.

Die nun einzeln vorliegenden Zellen Z1 bis Z5 liegen zwar nun getrennt vor, jedoch entsprechen diese nicht den an den entsprechenden Positionen in dem ursprünglichen zytologischen Präparat enthaltenen Zellen, da hier ja eine Überlappung der Zellplasmen vorlag, welche durch die einfache Trennung im Schritt S106 noch nicht berücksichtigt wurde. Aus diesem Grund legt das erfindungsgemäße Verfahren im Schritt S108 einen Bereich für benachbarte Zellen fest, in dem eine Überlappung der Zellplasmen der Zellen erwartet wird. Gemäß einem bevorzugten Ausführungsbeispiel wird zur Bestimmung dieses Bereichs ein Viereck aufgespannt, welches sich ausgehend von einem Zellkern zu einem ersten Einschnürungspunkt, von dort zu dem zweiten Zellkern, von dort zu dem zweiten Einschnürungspunkt und zurück zu dem ersten Zellkern erstreckt. In diesem Bereich wird erwartet, dass überlappendes Zellplasma vorliegt. In Fig. 1 sind die Überlappungsbereiche, die durch das gerade beschriebene Viereck definiert sind, mit U1 bis U4 bezeichnet.

Im Schritt S110 erfolgt eine Binarisierung der Überlappungsbereiche U1 bis U5, um die Bildpunkte jedes Überlappungsbereichs mittels eines Klassifikationsschrittes einer oder beiden betroffenen Zellen zuzuordnen.

Im Schritt S112 werden die im Schritt S106 gezeigten Zellen Z1 bis Z5 um die den jeweiligen Zellen zugeordneten Überlappungsbereiche erweitert und somit zu Einzelzellen, die den in der ursprünglichen zytologischen Probe enthaltenen Zellen entsprechen, vervollständigt. Alternativ kann im Schritt S112 eine Säuberung noch durchgeführt werden. Die so vorliegenden Einzelzellen werden im Bild noch etwas auseinandergezogen, um diese deutlich voneinander zu trennen.

Hinsichtlich des bevorzugten Ausführungsbeispiels wird darauf hingewiesen, dass die vorliegende Erfindung natürlich nicht hierauf beschränkt ist. Ganz allgemein ermöglicht die vorliegende Erfindung die Trennung eines Zellhaufens oder einer Zellgruppe, die zwei überlagerte Einzelzellen umfasst. Auch hier wird dann für jeden Zellkern festgestellt, welche relevanten Nachbarzellen in der Nähe liegen (Schritt S102). Anschließend werden zwischen den zwei benachbarten Zellkernen die Einschnürungspunkte erfasst (S104), welche als Markierung derjenigen Zellen dienen, an denen die Überlappung beendet ist. Die Zellen werden dann zunächst zwischen den Einschnürungspunkten getrennt und anschließend wird der Überlappungsbereich der Zellen, aufgespannt durch das Viereck zwischen den Einschnürungspunkten und den beiden Zellen, bestimmt. Die Bildpunkte des Überlappungsbereichs werden mittels eines Klassifikationsschrittes je einer oder beiden Zellen zugeordnet. Anschließend erfolgt, wie auch in Fig. 1 gezeigt, ein optionaler Säuberungsschritt.

Nachfolgend werden anhand der Fig. 2 bis 6 bevorzugte Ausführungsbeispiele zur Implementierung der anhand der Fig. 1 näher beschriebenen Schritte S102 bis S112 beschrieben.

Anhand der Fig. 2 wird nachfolgend die Bestimmung von benachbarten Zellkernen in einer Zellgruppe gemäß einem bevorzugten Ausführungsbeispiel näher erläutert. Betrachtet man eine Zellgruppe, die in Einzelzellen zu trennen ist, so stellt sich zunächst bei dem Heraustrennen eines Zellkerns die Frage welche anderen Zellkerne - und damit verbundene Zellplasmen - überhaupt "in der Nähe" angeordnet sind, so dass diese bei einer Teilung des gemeinsamen Plasmas berücksichtigt werden müssen. Der Ausdruck "in der Nähe" stellt eine Vereinfachung dar. Die Entscheidung für jeden der Zellkerne in einer Zellgruppe, ob er betrachtet werden muss oder nicht, trägt maßgeblich dazu bei, ob die herausgetrennte Zellplasmafläche korrekt ist.

Gemäß einem bevorzugten Ausführungsbeispiel wird die Frage, ob ein benachbarter Zellkern bei dem Heraustrennen eines betrachteten Zellkerns zu berücksichtigen ist, auf der Grundlage eines zwischen den zwei Zellkernen existierenden Abstandes, und ob die zwei Zellkerne in einem gemeinsamen Zellplasma angeordnet sind, beantwortet.

Beispielhaft seien die in Fig. 2A gezeigten Zellen Z1 und Z2 betrachtet die jeweils einen Zellkern ZK1 und ZK2 aufweisen. Ferner weisen die Zellen jeweils ein Zellplasma ZP1 und ZP2 auf, die einander überlappen, wodurch ein gemeinsames Zellplasma ZP gebildet ist. Der zulässige Abstand zwischen den zwei Zellkernen ZK1 und ZK2 liegt zwischen einem maximalen Abstand und einem minimalen Abstand. Der maximale Abstand und der minimale Abstand werden empirisch bestimmt. Bei einem bevorzugten Ausführungsbeispiel, bei dem die Informationen betreffend die einzelnen Zellen Z1 und Z2 in binären Bildern vorliegen, existieren für die einzelnen Zellkerne sogenannte Binärmasken und genauso existiert für das gemeinsame Zellplasma ZP eine Binärmaske. Gemäß einem bevorzugten Ausführungsbeispiel wird der euklidische Abstand zwischen dem Schwerpunkt der Binärmaske des herauszutrennenden Zellkerns ZK1 und dem Schwerpunkt der Binärmaske der verbleibenden Zellkerne, hier des Zellkerns ZK2 betrachtet, wobei der Abstand kleiner sein soll als 300 Pixel. Ebenso ist bei dem Ausführungsbeispiel der minimale Abstand festgelegt, der dadurch überprüft wird, dass von dem Abstand der Schwerpunkte jeweils der mittlere Abstand aller Randpunkte von dem Schwerpunkt beider Binärmasken abgezogen wird. Der sich hier ergebende Wert darf gemäß dem bevorzugten Ausführungsbeispiel nicht kleiner als 30 Pixel sein. Die gerade beschriebenen Pixelwerte gelten, ebenso wie die noch nachfolgend erwähnten Pixelwerte, für Bilder mit einer Auflösung von 1000 x 700 Pixel. Abhängig von der Kamera, dem Objektiv, etc. können Bilder mit anderen Auflösungen erzeugt werden, für die dann andere Pixelwerte gelten.

Liegt nun benachbart zu dem herauszutrennenden Zellkern ZK1 ein weiterer Zellkern ZK2, der die Erforderungen hinsichtlich des Abstandes erfüllt, so muss zusätzlich noch sichergestellt sein, dass diese beiden Zellkerne dem gemeinsamen Zellplasma ZP angehören. Um dies festzustellen, wird zwischen die Zellkerne ZK1 und ZK2 bzw. zwischen die Schwerpunkte der zugeordneten Binärmasken, eine Gerade G gelegt, die komplett innerhalb des gemeinsamen Zellplasmas des Zellhaufens liegen muss, wie dies in Fig. 2A gezeigt ist.

Sollte dies nicht der Fall sein, wird der Zellkern noch nicht verworfen, sondern zunächst wird eine sogenannte "indirekte Verbindung" geprüft. Die Überprüfung der indirekten Verbindung wird anhand der Fig. 2B und 2C beschrieben. Umgangssprachlich ausgedrückt ist eine "indirekte Verbindung" eine durch zwei Gerade G1 und G2 gebildete Verbindungslinie, wobei sich die Gerade G1 ausgehend von dem Zellkern ZK1 zu einem gemeinsamen Punkt, dem Knickpunkt K erstreckt, und wobei sich die zweite Gerade G2 ausgehend von dem zweiten Zellkern ZK2 zu dem gemeinsamen Knickpunkt K erstreckt, wie dies in Fig. 2B gezeigt ist. Geometrisch lässt sich diese Verbindung wie folgt beschreiben. Zwischen den Zellkernen ZK1 und ZK2 bzw. den Schwerpunkten der zugeordneten Binärmasken existiert eine Anzahl von Punkten, die jeweils den gleichen Abstand zu den Zellkernen besitzen. Diese Punkte liegen auf einer Geraden L (siehe Fig. 2C), welche senkrecht auf der Geraden G steht, welche die Zellkerne ZK1 und ZK2 verbindet, wobei die Gerade L mittig zwischen den zwei Zellkernen angeordnet ist. Alle auf der Geraden L liegenden Punkte werden nun einzeln betrachtet. Für jeden der betrachteten Punkte wird ausgehend von dem Zellkern ZK1 eine Gerade G1 und ausgehend von dem Zellkern ZK2 eine Gerade G2 zu dem untersuchten Punkt gebildet. Anschließend wird überprüft, ob beide Geraden G1 und G2 innerhalb des gemeinsamen Zellplasmas ZP liegen. Ist dies der Fall, so konnte über diese beiden Geraden G1 und G2 eine indirekte Verbindung zwischen den Schwerpunkten hergestellt werden.

Sollte die Betrachtung aller Punkte ergeben, dass mehrere dieser indirekten Verbindungen existieren, so wird jene gewählt, die den größten Winkel zwischen den zwei Geraden G1 und G2 aufweist, bzw. möglichst nahe an der Gerade G, welche die nächste Verbindung zwischen dem Zellkern darstellt, liegt, bzw. diejenige Verbindung, bei denen beide Geraden G1 und G2 möglichst kurz sind. Die gerade genannten drei Bedingungen sind äquivalent. Der am Ende gewählte Punkt ist der anhand der Fig. 2B bereits erwähnte Knickpunkt K.

Gemäß einem bevorzugten Ausführungsbeispiel ist ferner vorgesehen, dass der Knickpunkt K nur eine vorbestimmte Entfernung von dem Lotpunkt A, in dem die Gerade L die Gerade G schneidet, entfernt liegt. Bei einem bevorzugten Ausführungsbeispiel soll diese maximale Entfernung etwa 50 Pixel betragen.

Sind sowohl die Abstandsbedingung als auch die Verbindungsbedingung zweier Zellkerne erfüllt, so werden die zwei Zellkerne als benachbart zueinander angesehen. Sind beide oder eine der Bedingungen nicht erfüllt, so wird der ursprünglich herauszutrennende Zellkern ZK1 nicht weiter betrachtet und verworfen.

Obwohl anhand der Fig. 2A bis 2C ein Beispiel beschrieben wurde, ist die vorliegende Erfindung natürlich nicht hierauf beschränkt, insbesondere nicht auf die Verwendung von zwei Zellkernen. In der Fig. 2D sind fünf Zellkerne ZK1 bis ZK5 mit ihren entsprechenden direkten und indirekten Verbindungen gezeigt, die auf die oben beschriebene Art und Weise gefunden wurden. Bei dem in Fig. 2D gezeigten Beispielsbild handelt es sich um eine vergrößerte Darstellung der dem Schritt S102 zugeordneten Zellgruppe in Fig. 1.

In einer Situation, in der mehr als zwei Zellkerne vorliegen, und bei denen für zwei benachbarte Zellkerne eine indirekte Verbindung vorliegt, existiert der oben beschriebene Knickpunkt K. Für diesen Knickpunkt K werden nun die Abstände zu anderen Zellkernen betrachtet und bestimmt, ob einer dieser Abstände kleiner ist als der Abstand des Knickpunktes zu dem herauszutrennenden Zellkern, wie dies in Fig. 2E gezeigt ist. Hier wurde nach Feststellung der indirekten Verbindung zwischen dem Zellkern ZK1 und dem Zellkern ZK2 festgestellt, dass der Abstand X des Knickpunktes K zu dem Zellkern ZK3 kleiner ist als der Abstand des Knickpunktes K zu dem Zellkern ZK1. Der ursprünglich für die indirekte Betrachtung herangezogene Zellkern ZK2 wird daher für die weitere Heraustrennung des Zellkerns ZK1 verworfen, und an dessen Stelle tritt der Zellkern ZK3.

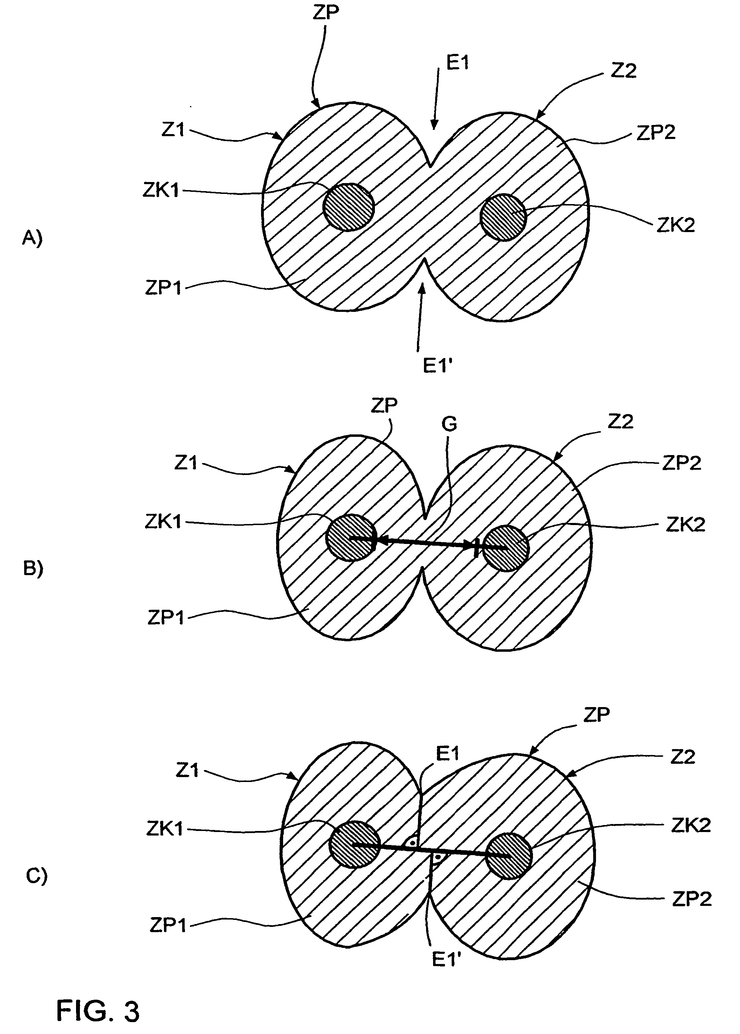

Nachfolgend wird anhand der Fig. 3A bis 3C ein bevorzugtes Ausführungsbeispiel zur Lokalisierung von Einschnürpunkten anhand zwei benachbarter Zellkerne näher erläutert. Nach dem Durchführen der anhand der Fig. 2 näher beschriebenen Schritte existieren nun für den herauszutrennenden Zellkern ZK1 eine Liste an Zellkernen, die bei dem Heraustrennen zu beachten sind. In dem vorliegenden Ausführungsbeispiel enthält diese Liste lediglich den benachbarten Zellkern ZK2. Stellt sich heraus, dass die Liste leer ist, also zu dem betrachteten, herauszutrennenden Zellkern ZK1 keine benachbarten Zellkerne existieren, so kann dieser Zellkern, und vor allem das zugehörige Zellplasma nicht herausgetrennt werden. Dieser Zellkern wird damit verworfen. Dies kann vorkommen, wenn der aktuelle Zellkern ZK1 auf dem Zellplasma eines Zellhaufens mit mehreren Zellkernen liegt, aber kein einziger dieser Zellkerne die oben beschriebenen Bedingungen hinsichtlich Abstand und Verbindung erfüllt. Zusätzlich zu den in der Liste eingetragenen Zellkernen ist dort auch die Position des Knickpunktes K angegeben, der für die kürzeste indirekte Verbindung zwischen dem in der Liste enthaltenen Zellkern und dem herauszutrennenden Zellkern aufgefunden wurde. Ist die Verbindung eine direkte Verbindung, so ist der Knickpunkt der Mittelpunkt zwischen den betrachteten Zellkernen, bei dem Ausführungsbeispiel wie es oben beschrieben wurde, zwischen dem herauszutrennenden Zellkern ZK1 und dem benachbarten Zellkern ZK2.

Basierend auf dem herauszulösenden Zellkern und dem benachbarten Zellkern sowie basierend auf dem Knickpunkt K wird nun nach Einschnürungspunkten E1, E1' (siehe Fig. 3A) gesucht. Gemäß einem bevorzugten Ausführungsbeispiel handelt es sich bei den Einschnürungspunkten um die engste Stelle des Zellplasmas ZP, welches die beiden Zellkernen ZK1 und ZK2 verbindet, wie dies in Fig. 3A durch die dort gezeigten Pfeile verdeutlicht ist.

Um die Einschnürungspunkte zu finden, wird durch den Knickpunkt K eine Gerade gelegt, welche parallel zu der Gerade G zwischen den zwei Zellkernen verläuft. Existiert eine direkte Verbindung zwischen den Zellkernen, so handelt es sich hierbei um die Gerade G.

Anschließend werden alle Randpunkte des zu der Zellgruppe gehörenden Plasmas, des gemeinsamen Zellplasmas ZP, betrachtet. Für jeden Randpunkt wird das Lot auf die durch den Knickpunkt gelegte Gerade gefällt, und anschließend werden die beiden Randpunkte gesucht, die die nachfolgenden Bedingungen erfüllen.

Die erste Bedingung besteht darin, dass die Punkte "zwischen" den Zellkernen liegen müssen, d. h. der Lotpunkt muss auf der Strecke zwischen den Zellkernen liegen. Dieser Bereich wird gemäß einem weiteren Ausführungsbeispiel noch eingeschränkt, indem von beiden Enden der Strecke zwischen den Zellkernen ein Teil von der Länge des jeweiligen mittleren Abstandes der Randpunkte Rₙ zu dem Schwerpunkt S des Zellkerns als "ungültig" erklärt wird, wie dies in Fig. 3B durch den der Gerade G zugeordneten Pfeil verdeutlicht ist.

Die zweite Bedingung ist, dass einer der gesuchten Punkte "links" und einer der gesuchten Punkte "rechts" der gewählten Gerade G liegen muss, wie dies in Fig. 3C verdeutlicht ist, in der der erste Punkt E1 oberhalb der Gerade G liegt, und der zweite Punkt E1' unterhalb der Gerade liegt. Bei der Lotbestimmung äußert sich dies in positiven bzw. negativen Vorzeichen.

Die letzte Bedingung besteht darin, dass auf beiden Seiten der Gerade G jeweils der Punkt mit dem kürzesten Lot gewählt wird.

Sind alle diese Bedingungen erfüllt, so sind die Einschnürungspunkte E1 und E1' zwischen dem herauszutrennenden Zellkern ZK1 und dem benachbarten Zellkern ZK2 festgelegt. Werden keine Randpunkte gefunden, die den oben genannten Bedingungen genügen, wird das Verfahren für den betrachteten Zellkern ZK1 abgebrochen, da keine für eine Teilung geeignete Position aufgefunden wurde.

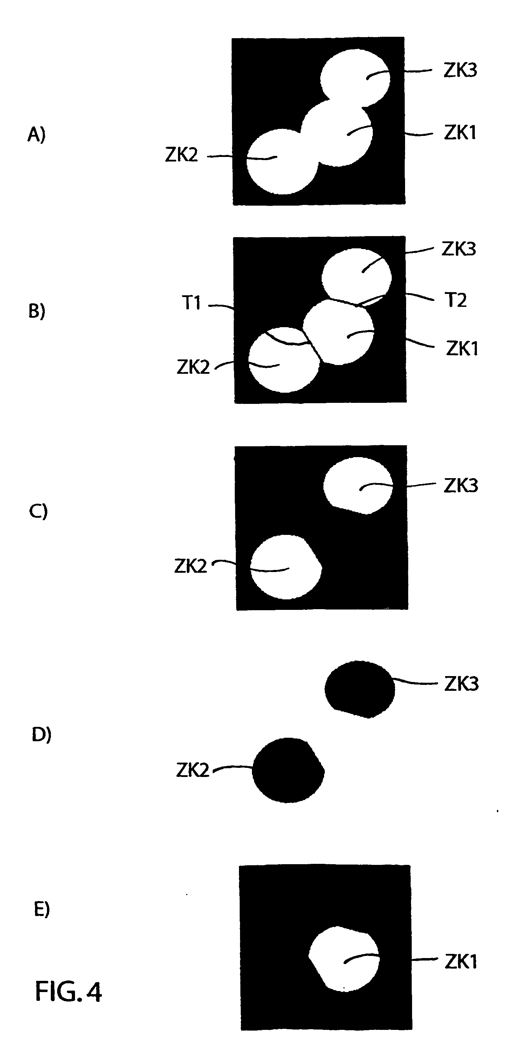

Nachdem die Einschnürungspunkte aufgefunden wurden, wird nun anhand der Fig. 4 ein bevorzugtes Ausführungsbeispiel zur Trennung des gemeinsamen Zellplasmas näher erläutert.

Die erfassten Einschnürungspunkte werden gemäß einem bevorzugten Ausführungsbeispiel der existierenden Liste von relevanten Zellkernen hinzugefügt. Zwischen jedem Paar, bestehend aus dem herauszutrennenden Zellkern und einem in der Liste abgelegten relevanten, weil benachbarten Zellkern, wird basierend auf den Einschnürpunkten eine Gerade zwischen denselben gezogen, und das gemeinsame Zellplasma der Zellgruppe wird an dieser Gerade "abgeschnitten". Dieser Schnitt wird durchgeführt, um eine grobe Grundlage für den herauszutrennenden Bereich des gemeinsamen Zellplasmas zu erhalten.

Gemäß einem bevorzugten Ausführungsbeispiel, welches die Informationen betreffend das gemeinsame Zellplasma und die Zellkerne in einer Binärmaske, die "weiße" und "schwarze" Pixel enthält, enthält, erfolgt das Abschneiden durch Einziehen einer "schwarzen" Linie zwischen den Einschnürungspunkten eines Paares, was für alle Einschnürungspunkte durchgeführt wird.

In Fig. 4A ist eine Binärmaske einer Zellgruppe gezeigt, wobei hier drei in der Binärmaske nicht zu erkennende Zellplasmen voneinander zu trennen sind. In Fig. 4A ist lediglich die Kontur des gemeinsamen Zellplasmas ZP zu erkennen. Der Einfachheit halber sind die einzelnen Abschnitte der Binärmaske in Fig. 4A mit ZK1, ZK2, ZK3 bezeichnet. Lediglich das Heraustrennen des Abschnitts ZK1 wird betrachtet. Der Algorithmus arbeitet derart, dass an den Einschnürpositionen Geraden T1, T2 gezogen werden, um die einzelnen Abschnitte voneinander zu trennen. Anschließend wird der herauszuschneidende Bereich gefüllt, so dass sich die in Fig. 4C gezeigte Binärmaske erstellt, die anschließend, wie in Fig. 4C gezeigt, invertiert wird. Eine Boolesche Schnittoperation der in Fig. 4D gezeigten Binärmaske mit der ursprünglichen Binärmaske gemäß Fig. 4A führt zu der Binärmaske in Fig. 4E, welche lediglich den aus der Zellgruppe herauszutrennenden Abschnitt des gemeinsamen Zellplasmas enthält.

Bevor nachfolgend ein möglicher Überlappungsbereich festgelegt wird, wird gemäß einem bevorzugten Ausführungsbeispiel der Abstand zwischen den zwei Einschnürungspunkten betrachtet: Sollte dieser Abstand einen vorbestimmten, empirisch festgelegten Grenzwert unterschreiten, beispielsweise 40 Pixel, so ist davon auszugehen, dass sich an dieser Schnittfläche die Zellplasmen der angrenzenden Zellkerne lediglich berührten, aber nicht wirklich überlappt hatten. Wird eine solche Situation festgestellt, so bedarf es keiner weiteren Bearbeitung, sondern der herausgetrennte Abschnitt zeigt tatsächlich die in der ursprünglichen Probe vorgelegene Zelle.

Ferner wird überprüft, ob der Abstand der Einschnürungspunkte einen Maximalwert nicht überschreitet, beispielsweise 350 Pixel. Wird eine Überschreitung des Maximalwertes festgestellt, so ist davon auszugehen, dass die Bestimmung der Einschnürungspunkte nicht korrekt verlaufen ist, da eine Überlappung dieser Länge unwahrscheinlich ist oder der Zellhaufen als unteilbar anzusehen ist, zumindest an dieser Stelle. In dieser Situation wird dann die Heraustrennung des interessierenden Zellkerns mit Zellplasma beendet.

Nachdem die grobe Grundlage des Zellplasmas aus der Zellgruppe herausgetrennt wurde, wird grundsätzlich angenommen, dass sich an jeder der Schnittflächen eine Überlappung von Zellplasmen verschiedener Zellen eingestellt hatte. Daher muss in einem nachfolgenden Schritt ein Bereich bestimmt werden, in dem nach dieser Überlappung der Plasmen zu suchen ist.

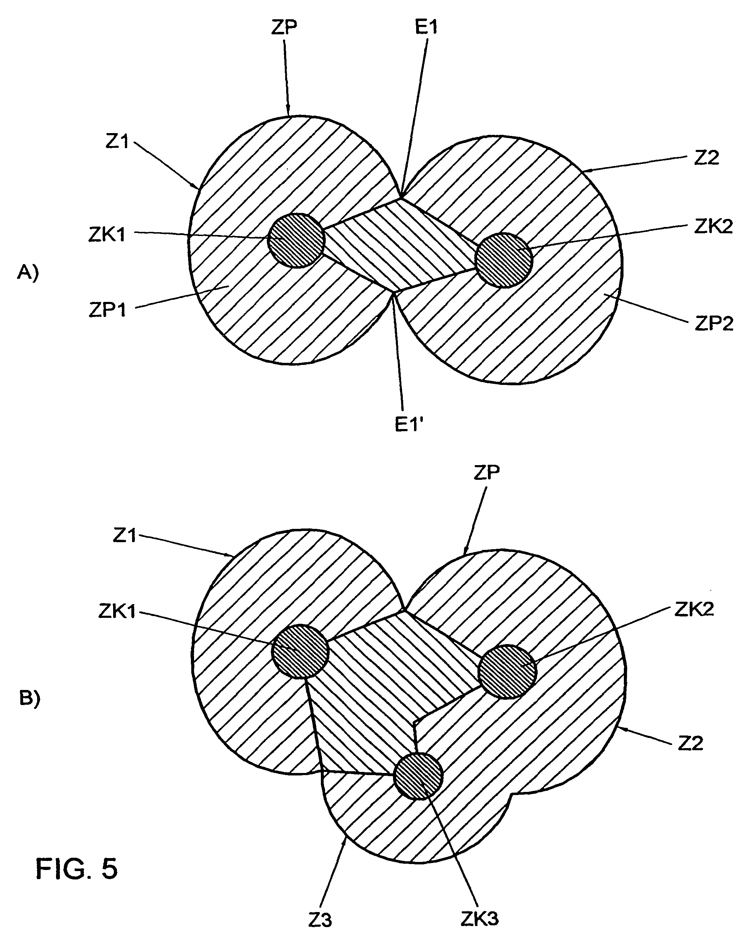

Anhand der Fig. 5A und 5B wird ein bevorzugtes Ausführungsbeispiel beschrieben, mittels dem ein interessierender Bereich festgelegt wird, in dem Überlappungen der Zellplasmen der in der ursprünglichen Probe enthaltenen Zellen erwartet werden.

Wie anhand der Fig. 5A gezeigt ist, wird mit den zwei Einschnürungspunkten E1, E1' und den zwei Zellkernen ZK1 und ZK2 ein Viereck gebildet, welches im Regelfall die Form einer Raute hat. Innerhalb dieses Vierecks wird die Überlappung der Zellplasmen in der ursprünglichen Probe erwartet. Die innere Fläche des Vierecks wird gemäß einem bevorzugten Ausführungsbeispiel wieder als Binärmaske repräsentiert, und mit der Binärmaske des gemeinsamen Zellplasmas der Zellgruppe auf Boolesche Weise geschnitten, da die Überlappung natürlich nur innerhalb des Plasmas liegen kann und daher kein Pixel außerhalb des Plasmas zu betrachten ist. Dieses ist erforderlich, da natürlich Teile des Vierecks auch außerhalb des Plasmas liegen können. Anschließend wird von der sich ergebenden Binärmaske die Binärmaske der beteiligten Zellkerne abgezogen, da auch die Bereiche der Zellkerne für die Erfassung der Überlappungsbereiche der Zellplasmen nicht herangezogen werden.

In einer Zellgruppe kann es vorkommen, dass für den herauszuschneidenden Zellkern mehrere benachbarte Zellkerne existieren. Für jeden werden die Einschnürungspunkte bestimmt und mögliche Überlappungsbereiche gebildet. Sollte es vorkommen, dass zwei oder mehr dieser möglichen Überlappungsbereiche sich schneiden, werden diese zu einer einzigen Binärmaske zusammengefasst und gemeinsam behandelt. Anhand der Fig. 5B ist eine solche Situation gezeigt, wobei der Überlappungsbereich schraffiert dargestellt ist.

Wie oben ausgeführt wurde, wird innerhalb des Überlappungsbereiches die Überlappung der Zellplasmen der in der ursprünglichen Probe enthaltenen Einzelzellen vermutet. Dies zeichnet sich beispielsweise in einem Durchlichtbild der Probe in der Regel durch einen dunkleren Farbwert aus, da zwei überlappende Plasmen dunkler erscheinen als ein Plasma. Diese Unterscheidung lässt sich am einfachsten mit einem Histogramm und einer geeigneten Schwellwertbestimmung lösen.

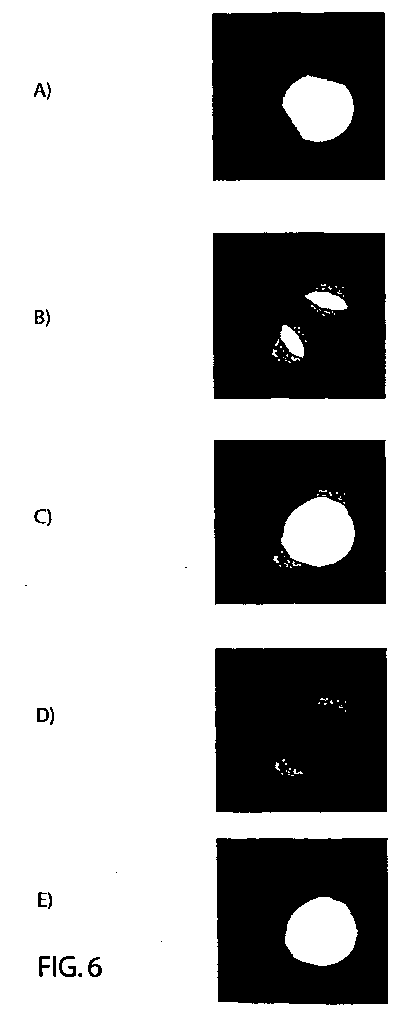

Gemäß einem bevorzugten Ausführungsbeispiel wird nun bezüglich des festgelegten Bereichs der Überlappung ein lokales Histogramm des erzeugten Bildes, beispielsweise des Durchlichtbildes, erstellt. Anschließend wird das Histogramm untersucht, um einen Schwellwert zu bestimmen und mit diesem das erzeugte Bild innerhalb der Bitmaske zu binarisieren. Diese Untersuchung kann beispielsweise unter Verwendung des Verfahrens von Otsu, welches näher von T. Lehmann, W. Oberschelp, E. Pelikan, und R. Repges in "Bildverarbeitung für die Medizin", Springer, Berlin 1997 beschrieben wird, durchgeführt werden. Hierdurch werden die dunkleren Pixel in der Überlappung weiß und die helleren Pixel in der Überlappung werden schwarz in der Binärmaske dargestellt. Dies ist in Fig. 6A und 6B verdeutlicht, wobei Fig. 6A die bereits vorab beschriebene Grobmaske für das Zellplasma zeigt. Fig. 6B zeigt die sich aufgrund der oben beschriebenen Schritte ergebende Überlappungs-Binärmaske.

Diese Überlappungs-Binärmaske wird mit der Binärmaske gemäß Fig. 6A vereinigt, woraus sich die in Fig. 6C gezeigte Binärmaske ergibt. Die noch am Rand befindlichen Artefakte werden gesäubert, so dass sich die abschließende Form, wie sie in Fig. 6E gezeigt ist ergibt.

Auf die oben beschriebene Art und Weise lassen sich somit mittels des erfindungsgemäßen Verfahrens Zellgruppen in einem Präparat in Einzelzellen zerlegen, so dass durch die Automatisierung an dieser Stelle eine Gesamtautomatisierung des Klassifizierungsverfahrens für zytologische Präparate erreicht wird.

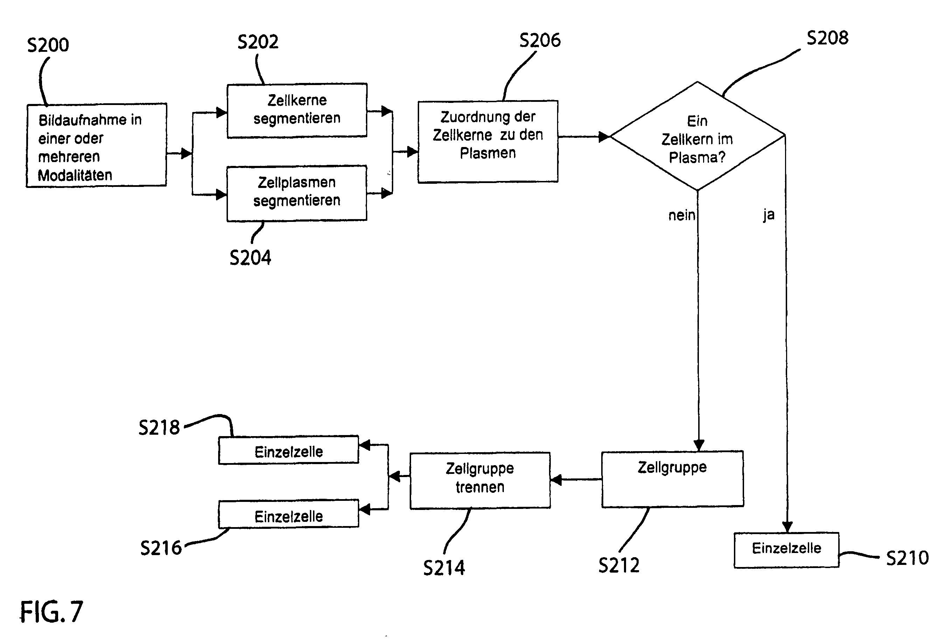

Wie oben erwähnt wurde, startet das erfindungsgemäße Verfahren mit einem Zellhaufen bzw. einer Zellgruppe, welcher aus einer Aufnahme einer zytologischen Probe erfasst wurde. Nachfolgend wird anhand der Fig. 7 ein Blockdiagramm eines weiteren bevorzugten Ausführungsbeispiels der vorliegenden Erfindung beschrieben, gemäß dem das Verfahren die erforderlichen Schritte zur Vorbereitung einer Zellgruppe umfasst.

Bei diesem Ausführungsbeispiel startet das Verfahren bei dem Schritt S200, in dem eine Bildaufnahme der zytologischen Probe in einer oder mehreren Modalitäten durchgeführt wird. Wie oben bereits erwähnt wurde, erfolgt entweder die Aufnahme eines Bildes mit einer Aufnahmemodalität, z. B. Durchlicht oder Fluoreszenz. Alternativ können auch mehrere, miteinander registrierte, multimodale Bilder erzeugt werden, beispielsweise durch Erzeugen von Bildern einer Probe in einer ersten Aufnahmemodalität und einer zweiten Aufnahmemodalität. Die erste Aufnahmemodalität kann beispielsweise eine Durchlichtaufnahmemodalität sein, und die zweite Aufnahmemodalität kann eine Fluoreszenzaufnahmemodalität sein. Alternativ können auch Fluoreszenzaufnahmemodalitäten mit unterschiedlichen Parametern eingesetzt werden.

Im nachfolgenden Schritt S202 werden die in der Aufnahme befindlichen Zellkerne erfasst und segmentiert, um eine Liste der in dem Bild bzw. in der Aufnahme enthaltenen Zellkerne zu erzeugen. Parallel hierzu erfolgt im Schritt S204 die Erfassung der in der Aufnahme enthaltenen Zellplasmen und eine Segmentierung derselben, um wiederum eine Liste zu erzeugen, die nun die Zellplasmen in der Aufnahme enthält. Es ist darauf hinzuweisen, dass bei der Verwendung von mehreren Bildern die Segmentierung von Zellkernen und die Segmentierung von Zellplasmen nicht in den gleichen Bildern erfolgen muss. Vorzugsweise wird die Segmentierung von Zellplasmen aufgrund von Durchlichtbildern erfolgen, wohingegen die Segmentierung von Zellkernen auf der Grundlage von Fluoreszenzbildern erfolgen kann. Nachdem die Zellkerne und Zellplasmen in der Probe erkannt wurden, werden im Schritt S206 die Zellkerne zu den Plasmen zugeordnet, über die erzeugten Listen. Anschließend wird im Schritt S208 überprüft ob einem Plasma lediglich ein einzelner Zellkern zugeordnet ist. Ist dies zutreffend, so liegt eine Einzelzelle vor, die keiner weiteren Segmentierung bedarf, und das Verfahren endet bei dem Schritt S210. Wird festgestellt, dass einem Plasma mehr als ein Zellkern zugeordnet sind, so geht das Verfahren zum Schritt S212 über, in dem festgestellt wird, dass eine Zellgruppe vorliegt. Diese Zellgruppe wird anschließend im Schritt S214 getrennt, so dass abschließend die Einzelzellen in den Schritten S216 und S218 zur Weiterverarbeitung vorliegen. Hinsichtlich der im Schritt S214 durchgeführten Schritte wird auf die obige Beschreibung des bevorzugten Ausführungsbeispiels zur Zellgruppentrennung verwiesen.

Nachfolgend wird ein bevorzugtes Ausführungsbeispiel zur Erkennung und Segmentierung der Zellplasmen und Zellkerne in der Aufnahme einer zytologischen Probe beschrieben.

Die Zellplasmasegmentierung erfolgt wahlweise in einem Durchlichtbild oder in einem Fluoreszenzbild der Probe. Die Zellplasmasegmentierung erfolgt unter Verwendung von Histogrammen. Hier wird ein vorbestimmter Schwellwert berechnet (z.B. durch das oben genannte Verfahren von Otsu), mit dem das Durchlichtbild binarisiert wird, um so Zellplasmen von dem helleren Hintergrund zu trennen. Für die Histogrammbildung und Schwellwertbildung sind verschiedene, im Stand der Technik gut bekannte Verfahren implementierbar.

Das durch den Histogramm-basierten Ansatz entstandene Binärbild der Aufnahme der Zelle wird nun untersucht, um Regionen in dem Binärbild, welche das Plasma, einschließlich Kern, einer Zelle wiedergeben oder einen Zellhaufen sich überlappender Zellen wiedergeben, zu bestimmen. Jeder unabhängige Bereich in dem Binärbild stellt eine eigene Region dar, und jeder eigenen Region, also jedem Plasma der Zelle bzw. jeder Fläche eines Zellhaufens sich überlappender Zellen wird ein Teilbild, beispielsweise in Form einer Binärmaske, zugeordnet.

Die Zellkernsegmentierung erfolgt ähnlich wie die Segmentierung der Zellplasmen, wahlweise in dem Durchlichtbild oder in dem Fluoreszenzbild. Auch hier wird der bekannte Histogrammbasierte Ansatz zur Erfassung von Zellkernen in der Aufnahme der zytologischen Probe verwendet, so dass sich für einzelne Zellkerne Teilbilder, beispielsweise in der Form von Binärmasken ergeben.

Die aus der Segmentierung der Zellplasmen resultierende Liste von Teilbildern (Binärmasken), wobei jedes Teilbild dem Plasma einer Zelle bzw. der Fläche eines Zellhaufens sich überlappender Zellen entspricht, und die aus der Segmentierung der Zellkerne resultierenden Teilbilder betreffender Zellkerne werden mittels einer einfachen Booleschen Operation verknüpft. Ist die Schnittmenge der Binärmasken des Zellkerns und der Binärmaske eines Zellplasmas nicht leer, so wird der Zellkern dem Zellplasma zugeordnet. Wird festgestellt, dass einem Zellplasma nur ein Zellkern zugeordnet ist, so handelt es sich hierbei bereits um fertig segmentierte Zellen mit einem Plasma und einem Zellkern. Sind einem Plasma zwei oder mehr Zellkerne zugeordnet, so liegt ein Zellhaufen oder eine Zellgruppe vor, die erfindungsgemäß zu trennen ist.

Bei einem Ausführungsbeispiel der vorliegenden Erfindung wird basierend auf den den erfassten Zellkernen zugeordneten Teilbildern eine Klassifizierung der Zellkerne durchgeführt, die einen Vergleich ausgewählter Parameter des Zellkerns mit vorbestimmten Parametern umfasst, um zu bestimmen ob ein erfasster Zellkern für eine Weiterverarbeitung geeignet ist.

Aufgrund der so vorbereiteten und aufbereiteten Aufnahme der zytologischen Probe führt das erfindungsgemäße Verfahren die Zerteilung der segmentierten Zellhaufen in Einzelzellen durch.

Bei der obigen Beschreibung des bevorzugten Ausführungsbeispiel wurde dargelegt, dass der Überlappungsbereich durch ein Viereck gebildet ist. Die vorliegende Erfindung ist nicht auf diese Ausgestaltung beschränkt, vielmehr kann der Überlappungsbereich durch eine Fläche beliebiger Form zwischen den Einschnürpunkten E1, E1' und den Zellkernen ZK1 und ZK2 aufgespannt sein.

## Patentansprüche

1. Verfahren zur Trennung einer in einem Bild einer Probe enthaltenen Zellgruppe in Einzelzellen für eine nachfolgende Klassifikation der Probe, wobei die Zellgruppe eine Mehrzahl einander überlappender Zellen aufweist, mit folgenden Schritten:
(a) Auswählen (S102) eines Zellkerns (ZK1) einer ersten Zelle (Z1), die aus der Zellgruppe herauszutrennen ist, wobei der Zellkern (ZK1) der ersten Zelle (Z1) benachbart zu einem Zellkern (Zk2) einer zweiten Zelle (Z2) angeordnet ist, wobei das Zellplasma der ersten Zelle (Z1) und das Zellplasma der zweiten Zelle (Z2) einander derart überlappen, dass ein gemeinsames Zellplasma (ZP) gebildet ist;
(b) Bestimmen (S104) einer Einschnürung des gemeinsamen Zellplasmas (ZP) zwischen dem Zellkern (ZK1) der ersten Zelle (Z1) und dem Zellkern (ZK2) der zweiten Zelle (Z2);
(c) Trennen des gemeinsamen Zellplasmas (ZP) an der Einschnürung;
(d) Festlegen eines Bereichs des gemeinsamen Zellplasmas (ZP), in dem die Überlappung der Zellplasmen der ersten Zelle (Z1) und der zweiten Zelle (Z2) erwartet wird;
(e) Klassifizieren des festgelegten Bereichs, um einzelne Abschnitte desselben zu dem Zellplasma der ersten Zelle (Z1) und/oder zu dem Zellplasma der zweiten Zelle (Z2) zuzuordnen; und
(f) Vervollständigen des im Schritt (c) erhaltenen Zellplasmas der ersten Zelle (Z1) basierend auf den klassifizierten Abschnitten.

2. Verfahren nach Anspruch 1, bei dem der Schritt (a) folgende Schritte umfasst:
(a.1.) Bestimmen eines Abstandes (G) zwischen dem Zellkern (ZK1) der ersten Zelle (Z1) und dem Zellkern (ZK2) der zweiten Zelle (Z2);
(a.2) Bestimmen, ob der Zellkern (ZK1) der ersten Zelle (Z1) und der Zellkern (ZK2) der zweiten Zelle (Z2) innerhalb des gemeinsamen Zellplasmas (ZP) angeordnet sind;
(a.3.) falls im Schritt (a.1) festgestellt wird, dass der Abstand außerhalb eines vorbestimmten Bereichs liegt und/oder falls im Schritt (a.2.) festgestellt wird, dass die Zellkerne nicht innerhalb des gemeinsamen Zellplasmas (ZP) angeordnet sind, Klassifizieren der Zellkerne (ZK1, ZK2) als nicht benachbart; und
(a.4.) falls im Schritt (a.1.) festgestellt wird, dass der Abstand innerhalb des vorbestimmten Bereichs liegt, und falls im Schritt (a.2) festgestellt wird, dass die Zellkerne innerhalb des gemeinsamen Zellplasmas (ZP) angeordnet sind, Klassifizieren der Zellkerne (ZK1, ZK2) als benachbart.

3. Verfahren nach Anspruch 2, bei dem im Schritt (a.2) festgestellt wird, ob der Zellkern (ZK1) der ersten Zelle (Z1) und der Zellkern (ZK2) der zweiten Zelle (Z2) durch eine Gerade (G) verbunden sind, die vollständig innerhalb des gemeinsamen Zellplasmas (ZP) verläuft, oder ob zwischen dem Zellkern (ZK1) der ersten Zelle (Z1) und dem Zellkern (ZK2) der zweiten Zelle (Z2) ein gemeinsamer Punkt (K) existiert, zu dem die Zellkerne (ZK1, ZK2) den gleichen Abstand haben und der in dem gemeinsamen Zellplasma (ZP) liegt.

4. Verfahren nach Anspruch 3, bei dem der Zellkern (ZK1) der ersten Zelle (Z1) und der Zellkern (ZK2) der zweiten Zelle (Z2) als nicht beabstandet klassifiziert werden, wenn eine weitere Zelle (Z3) mit einem Zellkern (ZK3) existiert, dessen Abstand zu dem gemeinsamen Punkt (K) kleiner ist als der Abstand des Zellkerns (ZK2) der zweiten Zelle (Z2) zu dem gemeinsamen Punkt (K).

5. Verfahren nach einem der Ansprüche 1 bis 4, bei dem der Schritt (c) folgenden Schritt umfasst, um zwei Einschnürungspunkte (E1, E1') des gemeinsamen Zellplasmas (ZP) zu bestimmen:
für alle Randpunkte des gemeinsamen Zellplasmas (ZP), die zwischen den Zellkernen (ZK1, ZK2) liegen, Bestimmen des Abstandes jedes Randpunktes zu einer festgelegten Geraden (L), und Auswählen der Randpunkte als Einschnürungspunkte (E1, E1'), die einen vorbestimmten Abstand zu der Gerade aufweisen.

6. Verfahren nach Anspruch 5, bei dem der vorbestimmte Abstand ein minimaler Abstand aller betrachteten Randpunkte ist.

7. Verfahren nach einem der Ansprüche 1 bis 6, bei dem die erste Zelle (Z1) im Schritt (c) entlang einer durch die Einschnürung festgelegten Trennlinie (T) aus der Zellgruppe herausgetrennt wird.

8. Verfahren nach einem der Ansprüche 1 bis 7, bei dem der Schritt (d) folgenden Schritt umfasst:
Festlegen des Bereichs, der zwischen den Zellkern (ZK1, ZK2) und den Einschnürungspunkten (E1, E1') liegt.

9. Verfahren nach einem der Ansprüche 1 bis 8, bei dem im Schritt (e) der festgelegte Bereich basierend auf der Transparenz des gemeinsamen Zellplasmas (ZP) in den einzelnen Abschnitten klassifiziert wird.

10. Verfahren nach einem der Ansprüche 1 bis 9, bei dem zumindest eine weitere Zelle in der Zellgruppe benachbart zu der ersten Zelle angeordnet ist, und bei dem die Schritte (b) bis (e) für die erste Zelle und die weitere Zelle ebenfalls durchgeführt werden.

11. Verfahren nach einem der Ansprüche 1 bis 10, bei dem das Bild der Probe durch eine Aufnahme der Probe in einer Durchlichtaufnahmemodalität erzeugt wird, oder bei dem das Bild eine Mehrzahl von Teilbildern umfasst, die miteinander registriert sind und bei gleichen oder unterschiedlichen Aufnahmemodalitäten erzeugt wurden.

12. Verfahren nach einem der Ansprüche 1 bis 11, bei dem das Verfahren vor dem Schritt (a) folgende Schritte umfasst:
Erfassen und Segmentieren (S102) von Zellkernen in einem Bild der Probe, um eine Liste von Zellkernen zu erzeugen;
Erfassen und Segmentieren (S204) von Zellplasmen in dem Bild, um eine Liste von Zellplasmen zu erzeugen;
Zuordnen der Zellkerne (S206) zu den zugehörigen Zellplasmen; und
Erkennen von Zellplasmen (S208), denen mehr als ein Zellkern zugeordnet ist, um eine Zellgruppe für eine nachfolgende Trennung festzulegen.

13. Verfahren nach Anspruch 12, bei dem der Schritt des Erfassens und Segmentierens von Zellkernen (S202) auf einem Bild der Probe basiert, das in einer Durchlichtaufnahmemodalität oder einer Fluoreszenzaufnahmemodalität erzeugt wurde, wobei Zellkerne in dem Bild basierend auf einem Histogramm und einem vorbestimmten Schwellenwert erfasst werden, und wobei jedem erfassten Zellkern ein Teilbild zugeordnet wird.

14. Verfahren nach Anspruch 13, bei dem basierend auf den den erfassten Zellkernen zugeordneten Teilbildern eine Klassifizierung der Zellkerne erfolgt, die einen Vergleich ausgewählter Parameter des Zellkerns mit vorbestimmten Parametern umfasst, um zu bestimmen ob ein erfaßter Zellkern für eine Weiterverarbeitung geeignet ist.

15. Verfahren nach Anspruch 13 oder 14, bei dem der Schritt des Suchens und Segmentierens von Zellplasmen (S204) auf einem Bild der Probe basiert, das in einer Durchlichtaufnahmemodalität oder in einer Fluoreszenzaufnahmemodalität erzeugt wurde, wobei Zellplasmen in dem Bild basierend auf einer Kantenerkennung oder basierend auf einem Histogramm und einem vorbestimmten Schwellwert erfasst werden, wobei jedem erfassten Zellplasma ein Teilbild zugeordnet wird.

16. Verfahren nach einem der Ansprüche 12 bis 15, bei dem das Bild der Probe bei der Suche und Segmentierung von Zellkernen und Zellplasmen binarisiert wird, und wobei die Teilbilder durch Binärmasken gebildet sind.

17. Verfahren nach einem der Ansprüche 13 bis 16, bei dem der Schritt des Zuordnens von Zellkernen zu zugehörigen Zellplasmen (S206) und des Erkennens von Zellgruppen (S208) den Vergleich der für die Zellkerne und die Zellplasmen erzeugten Teilbilder umfasst, wobei abhängig von dem Vergleich ein oder mehrere Zellkerne zu einem Zellplasma zugeordnet werden, wobei die einander zugeordneten Teilbilder einer ersten Gruppe, die Teilbilder eines Zellplasmas und eines Zellkerns enthalten, und einer zweiten Gruppe zugeordnet werden, die Teilbilder eines Zellplasmas und einer Mehrzahl von Zellkernen enthalten, wobei die Teilbilder der zweiten Gruppe eine Zellgruppe anzeigen, die der weiteren Verarbeitung zugrunde gelegt wird.

## Claims

1. Method for separating a cell group contained in an image of a sample into individual cells for a subsequent classification of the sample, wherein the cell group comprises a plurality of mutually overlapping cells, the method comprising:
(a) selecting (S102) a cell nucleus (ZK1) of a first cell (Z1) which is to be separated from the cell group, wherein the cell nucleus (ZK1) of the first cell (Z1) is located adjacent to a cell nucleus (ZK2) of a second cell (Z2), wherein the cell plasma of the first cell (Z1) and the cell plasma of the second cell (Z2) overlap each other such that a common cell plasma (ZP) is formed;
(b) determining (S104) a contraction of the common cell plasma (ZP) between the cell nucleus (ZK1) of the first cell (Z1) and the cell nucleus (ZK2) of the second cell (Z2);
(c) separating the common cell plasma (ZP) at the contraction;
(d) determining an area of the common cell plasma (ZP) in which the overlapping of the cell plasmas of the first cell (Z1) and the second cell (Z2) is expected;
(e) classifying the determined area to associate individual portions of the same with the cell plasma of the first cell (Z1) and/or the cell plasma of the second cell (Z2); and
(f) completing the cell plasma of the first cell (Z1) obtained in step (c) based on the classified portions.

2. Method of claim 1, wherein step (a) comprises the following steps:
(a.1.) determining a distance (G) between the cell nucleus (ZK1) of the first cell (Z1) and the cell nucleus (ZK2) of the second cell (Z2);
(a.2.) determining whether the cell nucleus (ZK1) of the first cell (Z1) and the cell nucleus (ZK2) of the second cell (Z2) are located within the common cell plasma (ZP);
(a.3.) if, in step (a.1.), the distance is detected to be outside a predetermined range, and/or if, in step (a.2.), the cell nuclei are detected not to be located within the common cell plasma (ZP), classifying the cell nuclei (ZK1, ZK2) as not adjacent; and
(a.4.) if, in step (a.1.), the distance is detected to be within the predetermined range, and if, in step (a.2.), the cell nuclei are detected to be located within the common cell plasma (ZP), classifying the cell nuclei (ZK1, ZK2) as adjacent.

3. Method of claim 2, wherein, in step (a.2.), it is detected whether the cell nucleus (ZK1) of the first cell (Z1) and the cell nucleus (ZK2) of the second cell (Z2) are connected by a straight line (G) running completely within the common cell plasma (ZP), or whether, between the cell nucleus (ZK1) of the first cell (Z1) and the cell nucleus (ZK2) of the second cell (Z2), there is a common point (K) to which the cell nuclei (ZK1, ZK2) have the same distance and which is located in the common cell plasma (ZP).

4. Method of claim 3, wherein the cell nucleus (ZK1) of the first cell (Z1) and the cell nucleus (ZK2) of the second cell (Z2) are classified as not adjacent, if another cell (Z3) with a cell nucleus (ZK3) exists whose distance to the common point (K) is smaller than the distance of the cell nucleus (ZK2) of the second cell (Z2) to the common point (K).

5. Method according to one of claims 1 to 4, wherein step (c) includes the following step in order to determine two contraction points (E1, E1') of the common cell plasma (ZP):
for all boundary points of the common cell plasma (ZP) located between the cell nuclei (ZK1, ZK2), determining the distance of each boundary point to a determined straight line (L) and selecting those boundary points as contraction points (E1, E1') which have a predetermined distance to the straight line.

6. Method of claim 5, wherein the predetermined distance is a minimum distance of all examined boundary points.

7. Method according to one of claims 1 to 6, wherein, in step (c), the first cell (Z1) is separated from the cell group along a separating line (T) determined by the contraction.

8. Method according to one of claims 1 to 7, wherein step (d) includes the following step:
determining the area located between the cell nuclei (ZK1, ZK2) and the contraction points (E1, E1').

9. Method according to one of claims 1 to 8, wherein, in step (e), the determined area is classified based on the transparency of the common cell plasma (ZP) in the individual portions.

10. Method according to one of claims 1 to 9, wherein at least one further cell in the cell group is located adjacent to the first cell, and wherein the steps (b) to (e) are also performed for the first cell and the further cell.

11. Method according to one of claims 1 to 10, wherein the image of the sample is generated by a picture of the sample in a transmitted light capturing modality, or wherein the image includes a plurality of sub-images which are registered with each other and which were generated in the same or in different capturing modalities.

12. Method according to one of claims 1 to 11, wherein the method includes the following steps prior to step (a):
detecting and segmenting (S102) cell nuclei in an image of the sample to generate a list of cell nuclei;
detecting and segmenting (S204) cell plasmas in the image to generate a list of cell plasmas;
associating the cell nuclei (S206) with the pertinent cell plasmas; and
detecting cell plasmas (S208) associated with more than one cell nucleus to determine a cell group for a subsequent separation.

13. Method of claim 12, wherein the step of detecting and segmenting cell nuclei (S202) is based on an image of the sample generated in a transmitted light capturing modality or a fluorescence capturing modality, wherein cell nuclei in the image are detected based on a histogram and a predetermined threshold value, and wherein a sub-image is associated with each detected cell nucleus.

14. Method of claim 13, wherein, based on the sub-images associated with the detected cell nuclei, a classification of the cell nuclei is performed which includes a comparison of selected parameters of the cell nucleus with predetermined parameters in order to determine whether a detected cell nucleus is suitable for further processing.

15. Method of claim 13 or 14, wherein the step of searching for and segmenting cell plasmas (S204) is based on an image of the sample generated in a transmitted light capturing modality or in a fluorescence capturing modality, wherein cell plasmas in the image are detected based on an edge detection or based on a histogram and a predetermined threshold value, wherein a sub-image is associated with each detected cell plasma.

16. Method according to one of claims 12 to 15, wherein the image of the sample is binarized in the search for and segmentation of cell nuclei and cell plasmas, and wherein the sub-images are formed by binary masks.

17. Method according to one of claims 13 to 16, wherein the step of associating cell nuclei with pertinent cell plasmas (S206) and detecting cell groups (S208) includes the comparison of the sub-images generated for the cell nuclei and the cell plasmas, wherein, depending on the comparison, one or more cell nuclei are associated with a cell plasma, wherein the sub-images associated with each other are associated with a first group containing sub-images of a cell plasma and one cell nucleus and a second group containing sub-images of a cell plasma and a plurality of cell nuclei, wherein the sub-images of the second group indicate a cell group on which the further processing is based.

## Revendications

1. Procédé permettant de séparer un groupe de cellules contenu dans une image d'un échantillon en cellules individuelles, pour une classification successive de l'échantillon, le groupe de cellules présentant une pluralité de cellules se recouvrant, aux étapes suivantes consistant à :
(a) sélectionner (S102) un noyau (ZK1) d'une première cellule (Z1) qui doit être séparée du groupe des cellules, le noyau (ZK1) de la première cellule (Z1) étant disposé adjacent à un noyau (ZK2) de la deuxième cellule (Z2), le plasma de la première cellule (Z1) et le plasma de la deuxième cellule (Z2) se recouvrant de sorte que soit formé un plasma de cellule commun (ZP) ;
(b) déterminer (S104) une constriction du plasma de cellule commun (ZP) entre le noyau (ZK1) de la première cellule (Z1) et le noyau (ZK2) de la deuxième cellule (Z2) ;
(c) séparer le plasma de cellule commun (ZP) à la constriction ;
(d) fixer une zone du plasma de cellule commun (ZP) dans laquelle est attendu le recouvrement des plasmas de la première cellule (Z1) et de la deuxième cellule (Z2) ;
(e) classifier la zone fixée, pour associer des segments individuels de cette dernière au plasma de la première cellule (Z1) et/ou au plasma de la deuxième cellule (Z2) ; et
(f) compléter le plasma de la première cellule (Z1) obtenu à l'étape (c) sur base des segments classifiés.

2. Procédé selon la revendication 1, dans lequel l'étape (a) comprend les étapes suivantes consistant à :
(a.1) déterminer une distance (G) entre le noyau (ZK1) de la première cellule (Z1) et le noyau (ZK2) de la deuxième cellule (Z2) ;
(a.2) déterminer si le noyau (ZK1) de la première cellule (Z1) et le noyau (ZK2) de la deuxième cellule (Z2) sont disposés dans le plasma de cellule commun (ZF) ;
(a.3.) s'il est constaté à l'étape (a.1) que la distance se situe en dehors d'une zone prédéterminée et/ou s'il est constaté à l'étape (a.2.) que les noyaux de cellule ne sont pas disposés dans le plasma de cellule commun (ZP), classifier les noyaux de cellule (ZK1, 2K2) comme n'étant pas voisins ; et
(a.4.) s'il est constaté à l'étape (a.1.) que la distance se situe dans la zone prédéterminée, et qu'il est constaté à l'étape (a.2) que les noyaux de cellule sont disposés dans le plasma de cellule commun (ZP), classifier les noyaux de cellule (ZK1, ZK2) comme étant voisins.

3. Procédé selon la revendication 2, dans lequel est constaté à l'étape (a.2) si le noyau (ZK1) de la première cellule (Z1) et le noyau (ZK2) de la deuxième cellule (Z2) sont reliés par une ligne droite (G) qui s'étend entièrement dans le plasma de cellule commun (ZP), ou s'il existe entre le noyau (ZK1) de la première cellule (Z1) et le noyau (ZK2) de la deuxième cellule (Z2) un point commun (K) par rapport auquel les noyaux de cellule (ZK1, 2K2) présentent la même distance et qui se situe dans le plasma de cellules commun (2P).

4. Procédé selon la revendication 3, dans lequel le noyau (ZK1) de la première cellule (Z1) et le noyau (ZK2) de la deuxième cellule (Z2) sont classifiés comme étant non distants lorsqu'il existe une autre cellule (Z3) avec un noyau de cellule (ZK3) dont la distance par rapport au point commun (K) est inférieure à la distance entre le noyau (ZK2) de la deuxième cellule (Z2) et le point commun (K).

5. Procédé selon l'une des revendications 1 à 4, dans lequel l'étape (c) comprend l'étape suivante, pour déterminer deux points de constriction (E1, E1') du plasma de cellule commun (ZP), consistant à :
pour tous les points de bordure du plasma de cellule commun (ZF) qui se situent entre les noyaux de cellule (ZK1, ZK2), déterminer la distance de chaque point de bordure par rapport à une ligne droite (L) fixée, et sélectionner les points de bordure comme points de constriction (E1, E1') qui présentent une distance prédéterminée par rapport à la ligne droite.

6. Procédé selon la revendication 5, dans lequel la distance prédéterminée est une distance minimale de tous les points de bordure considérés.

7. Procédé selon l'une des revendications 1 à 6, dans lequel la première cellule (Z1) est séparée, à l'étape (c), selon une ligne de séparation (T) fixée par la constriction, du groupe des cellules.

8. Procédé selon l'une des revendications 1 à 7, dans lequel l'étape (d) comprend l'étape suivante consistant à :
fixer la zone qui se situe entre les noyaux de cellule (ZK1, ZK2) et les points de constriction (E1, E1').

9. Procédé selon l'une des revendications 1 à 8, dans lequel la zone fixée est classifiée, à l'étape (e), sur base de la transparence du plasma de cellule commun (ZP) dans les différents segments.

10. Procédé selon l'une des revendications 1 à 9, dans lequel au moins une autre cellule dans le groupe de cellules est disposée adjacente à la première cellule, et dans lequel les étapes (b) à (e) sont également réalisées pour la première cellule et l'autre cellule.

11. Procédé selon l'un des revendications 1 à 10, dans lequel l'image de l'échantillon est générée par une prise de vue de l'échantillon selon une modalité de prise de vue par transparence, ou dans lequel l'image comporte une pluralité d'images partielles qui sont en registre l'une avec l'autre et qui ont été générées selon des modalités de prise de vue identiques ou différentes.

12. Procédé selon l'une des revendications 1 à 11, dans lequel le procédé comprend, avant l'étape (a), les étapes suivantes consistant à :
détecter et segmenter (S102) les noyaux de cellule dans une image de l'échantillon, pour générer une liste de noyaux de cellule ;
détecter et segmenter les plasmas de cellule (S204) dans l'image, pour générer une liste de plasmas de cellule ;
associer les noyaux de cellule (S206) aux plasmas de cellule y relatifs ; et
identifier les plasmas de cellule (S208) auxquels est associé plus d'un noyau de cellule, pour établir un groupe de cellules pour une séparation successive.

13. Procédé selon la revendication 12, dans lequel l'étape de détection et de segmentation de noyaux de cellule (S202) se base sur une image de l'échantillon qui a été générée selon une modalité de prise de vue par transparence ou une modalité de prise de vue à fluorescence, les noyaux de cellule dans l'image étant détectés sur base d'un histogramme et d'une valeur de seuil prédéterminée, et à chaque noyau de cellule détecté étant associée une image partielle.

14. Procédé selon la revendication 13, dans lequel a lieu, sur base des images partielles associées aux noyaux de cellule détectés, une classification des noyaux de cellule qui comprend une comparaison de paramètres sélectionnés du noyau de cellule à paramètres prédéterminés, pour déterminer si un noyau de cellule détecté convient pour un traitement successif.

15. Procédé selon la revendication 13 ou 14, dans lequel l'étape de recherche et de segmentation de plasmas de cellule (S204) se base sur une image de l'échantillon qui a été générée selon une modalité de prise de vue par transparence ou une modalité de prise de vue à fluorescence, les plasmas de cellule dans l'image étant détectés sur base d'une identification de bord ou sur base d'un histogramme et d'une valeur de seuil prédéterminée, à chaque plasma de cellule détecté étant associée une image partielle.

16. Procédé selon l'une des revendications 12 à 15, dans lequel l'image de l'échantillon est binarisée lors de la recherche et de la segmentation de noyaux de cellule et de plasmas de cellule, et les images partielles étant formées par des masques binaires.

17. Procédé selon l'une des revendications 13 à 16, dans lequel l'étape d'association de noyaux de cellule à des plasmas de cellule y relatifs (S206) et de l'identification de groupes de cellules (S208) comprend la comparaison des images partielles générées pour les noyaux de cellule et les plasmas de cellule, un ou plusieurs noyaux de cellule étant associés, en fonction de la comparaison, à un plasma de cellule, les images partielles associées l'une à l'autre contenant un premier groupe que contient des images partielles d'un plasma de cellule et d'un noyau de cellule, et à un deuxième groupe contenant des images partielles d'un plasma de cellule et une pluralité de noyaux de cellule, les images partielles du deuxième groupe indiquant un groupe de cellules qui sert de base au traitement ultérieur.
